# EUROPEAN PATENT SPECIFICATION

(11) **EP 2 609 218 B1**
(45) Date of publication and mention of the grant of the patent: **23.08.2017**
(21) Application number: 11819430.7
(22) Date of filing: 25.08.2011
(51) Int. Cl.: C12Q 1/68, A61K 49/16, A61K 48/00, A61P 9/10, A61P 13/12

(54) **METHODS AND KITS FOR PREDICTING THE RISK OF DIABETIC KIDNEY COMPLICATIONS USING GENETIC MARKERS AND ARRAYS**
VERFAHREN UND KITS ZUR VORHERSAGE DES RISIKOS VON NIEREN-KOMPLIKATIONEN BEI DIABETES MITHILFE GENETISCHER MARKER UND ARRAYS
MÉTHODES ET TROUSSES POUR LA PRÉDICTION DU RISQUE DE COMPLICATIONS ASSOCIÉES AU DIABÈTE RÉNAL À L'AIDE DE MARQUEURS ET DE PUCES GÉNÉTIQUES

(30) Priority: 25.08.2010 US 376847 P
(43) Date of publication of application: 03.07.2013
(73) Proprietor: The Chinese University of Hong Kong, Shatin, N.T. Hong Kong (CN)
(72) Inventor: MA, Ronald Ching Wan, Hong Kong (CN); SO, Wing Yee, Kowloon Hong Kong (CN); NG, Maggie Chor Yin, Hong Kong (CN); CHAN, Juliana Chung Ngor, Tai Po, NT Hong Kong (CN)
(74) Representative: Gill Jennings & Every LLP
(86) International application number: PCT/CN2011/078910
(87) International publication number: WO 2012/025055

(56) References cited:
- CN-A- 1 515 686
- S.-I. ARAKI: "Identification of a Common Risk Haplotype for Diabetic Nephropathy at the Protein Kinase C- 1 (PRKCB1) Gene Locus", JOURNAL OF THE AMERICAN SOCIETY OF NEPHROLOGY, vol. 14, no. 8, 1 August 2003 (2003-08-01) , pages 2015-2024, XP055081270, ISSN: 1046-6673, DOI: 10.1097/01.ASN.0000077347.27669.5C
- ARAKI SHIN-ICHI ET AL: "The association between polymorphisms of the PKC-beta gene and diabetic kidney disease in Japanese type 2 diabetes mellitus", DIABETES, AMERICAN DIABETES ASSOCIATION, US, vol. 55, no. Suppl. 1, 1 June 2006 (2006-06-01), page A176, XP009174932, ISSN: 0012-1797
- S.-I. ARAKI: "Polymorphisms of the Protein Kinase C- Gene (PRKCB1) Accelerate Kidney Disease in Type 2 Diabetes Without Overt Proteinuria", DIABETES CARE, vol. 29, no. 4, 1 April 2006 (2006-04-01), pages 864-868, XP055092250, ISSN: 0149-5992, DOI: 10.2337/diacare.29.04.06.dc05-1723
- Y. IKEDA ET AL: "Polymorphisms in the 5'-upstream region of the PKCbeta gene in Japanese patients with Type 2 diabetes", DIABETIC MEDICINE, vol. 21, no. 10, 1 October 2004 (2004-10-01), pages 1113-1120, XP55092283, ISSN: 0742-3071, DOI: 10.1111/j.1464-5491.2004.01304.x
- ERIC R. GAMAZON ET AL: "Comprehensive Survey of SNPs in the Affymetrix Exon Array Using the 1000 Genomes Dataset", PLOS ONE, vol. 5, no. 2, 23 February 2010 (2010-02-23), page e9366, XP055092619, DOI: 10.1371/journal.pone.0009366
- Snpdev: "Submitted SNP(ss) Details: ss14262562 , rs3760106", , 5 October 2003 (2003-10-05), XP055092523, Retrieved from the Internet: URL:http://www.ncbi.nlm.nih.gov/projects/S NP/snp_ss.cgi?subsnp_id=14262562 [retrieved on 2013-12-10]
- Snpdev: "Submitted SNP(ss) Details: ss1440834 , rs2575390", , 21 September 2002 (2002-09-21), XP055092514, Retrieved from the Internet: URL:http://www.ncbi.nlm.nih.gov/projects/S NP/snp_ss.cgi?subsnp_id=1440834 [retrieved on 2013-12-10]
- Snpdev: "Submitted SNP(ss) Details: ss11187802 , rs7404928", , 3 July 2003 (2003-07-03), XP055092508, Retrieved from the Internet: URL:http://www.ncbi.nlm.nih.gov/SNP/snp_ss .cgi?subsnp_id=11187802 [retrieved on 2013-12-10]
- Snpdev: "Submitted SNP(ss) Details: ss6606722 , rs4787733", , 12 February 2003 (2003-02-12), XP055092512, Retrieved from the Internet: URL:http://www.ncbi.nlm.nih.gov/projects/S NP/snp_ss.cgi?subsnp_id=6606722 [retrieved on 2013-12-10]
- MA R.C.W. ET AL.: 'Genetic Variants of the Protein Kinase C- beta1 Gene and Development of End-Stage Renal Disease in Patients With Type 2 Diabetes' JAMA vol. 304, no. 8, 25 August 2010, pages 881 - 889, XP055081265
- ARAKI S.-I. ET AL.: 'Identification of a Common Risk Haplotype for Diabetic ephropathy at the Protein Kinase C-1 (PRKCBl ) Gene Locus' JOURNAL OF THE AMERICAN SOCIETY OF NEPHROLOGY vol. 14, 2003, pages 2015 - 2024, XP055081270
- "Affymetrix Genome-Wide Human SNP 5.0 Array", GEO, 2007, XP002711695, [retrieved on 2008-04-30]
- AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet .pdf [retrieved on 2009-04-10]

## Description

### FIELD OF THE INVENTION

The present invention relates to methods, kits and arrays for diagnosing or detecting a subject's genetic susceptibility for a disease, condition, or disorder in a subject, in particular for diabetic kidney complications including end stage renal disease (ESRD).

### BACKGROUND OF THE INVENTION

Diabetic nephropathy is a leading cause of morbidity and mortality in diabetic patients. With the rising epidemic of diabetes in both developing and developed countries, diabetes is now the major contributing cause of end stage renal disease (ESRD). Asia is in the midst of an epidemic of type 2 diabetes (non-insulin dependent diabetes mellitus)^{1 2 3}. A recent large-scale epidemiology survey estimated that there are 94.2 million adults affected by diabetes in China, with another 148.2 million with prediabetes ⁴. Renal failure or ESRD is an important cause of mortality among patients with type 2 diabetes ^{5 6}. Asian populations appear to be particularly at risk of diabetic kidney disease (DKD). In the MAP study, microalbuminuria was present in 40% and macroalbuminuria in 20% of Asian patients with type 2 diabetes and hypertension ⁷. Progression to ESRD can be effectively reduced by aggressive reduction of multiple targets including blood pressure, glucose control, lipid parameters as well as agents which modulate the renin-angiotensin system. However, it is often not possible to predict which subjects with diabetes are at increased risk of developing renal complications, especially the ESRD.

Protein kinase C (PKC)-β is an important molecule involved in cell signaling, and has been implicated in the development of diabetic microvascular complications as well as diabetic cardiomyopathy ^{14 15 16 17}. Pharmacological inhibition of PKC-β was found to be effective in normalizing haemodynamic changes, extracellular matrix (ECM) accumulation, histological features of glomerular damage as well as reduced albuminuria in diabetic animal model ^{18 19 20}. Mice that lack PKC-β are protected from glomerular hypertrophy, oxidative stress and albuminuria when exposed to hyperglycaemia ²¹. In randomized clinical trials, inhibitor of PKC-β decreased loss of glomerular filtration rate and proteinuria in diabetic patients who are optimally treated with ACEI or ARB ²². These data support an important role of PKC-β in the pathogenesis of DKD. Both isoforms of PKC-β, PKC-βI and PKC-βII, are encoded by the PKC-β1 gene (*PRKCB*) on the short arm of chromosome 16. Type 1 diabetic patients (insulin dependent diabetes mellitus) carrying 2 single nucleotide polymorphism (SNP)s in the promoter region of the *PRKCB* had increased risk of nephropathy ^{23 24}.

In Diabetes, American Diabetes Association US, Vol.55, No. Suppl. 1, 1 June 2006, Page A176, Shin-Ichi Araki *et al* describe the association between polymorphisms of the PKC-beta gene and diabetic kidney disease in Japanese Type 2 Diabetes Mellitus.

### SUMMARY

The present inventors have surprisingly discovered that certain single nucleotide polymorphisms (SNPs) within the PKC-β1 (*PRKCB*) gene and related regulatory regions are associated with the development of diabetic kidney complications comprising end stage renal disease (ESRD) due to type 2 diabetes, ESRD due to hypertension in type 2 diabetes and ESRD due to type 1 diabetes. Therefore, the SNPs are useful for methods and kits of diagnosing a genetic predisposition for the development of the diseases in individuals.

Accordingly, one aspect disclosed herein provides a method for diagnosing a genetic predisposition in a subject for the diseases, disorders or conditions selected from the group consisting of end stage renal disease (ESRD) due to type 2 diabetes, ESRD due to hypertension in type 2 diabetes and ESRD due to type 1 diabetes, comprising analyzing at least one polynucleotide to detect (i) a haplotype consisting of three variants or single nucleotide polymorphisms (SNPs) which are T allele at rs3760106,TT genotype at rs7404928, and A allele at rs4787733; and/or (ii) a haplotype consisting of four variants or single nucleotide polymorphisms (SNPs) which are T allele at rs3760106, G allele at rs2575390, TT genotype at rs7404928 and A allele at rs4787733, wherein the presence of either or both haplotypes indicates the subject is suffering from, at risk for, or suspected of suffering from said diseases, disorders or conditions.

Another aspect disclosed herein provides a kit or array for use in diagnosing a genetic predisposition in a subject for the diseases, disorders or conditions selected from the group consisting of end stage renal disease (ESRD) due to type 2 diabetes, ESRD due to hypertension in type 2 diabetes and ESRD due to type 1 diabetes. The kit or array consists of reagents for detecting (i) a haplotype consisting of three variants or single nucleotide polymorphisms (SNPs) which are T allele at rs3760106, TT genotype at rs7404928, and A allele at rs4787733; and/or (ii) a haplotype consisting of four variants or single nucleotide polymorphisms (SNPs) which are T allele at rs3760106, G allele at rs2575390, TT genotype at rs7404928 and A allele at rs4787733 in a sample containing at least one polynucleotide obtained from the subject.

### BRIEF DESCRIPTION OF THE DRAWINGS

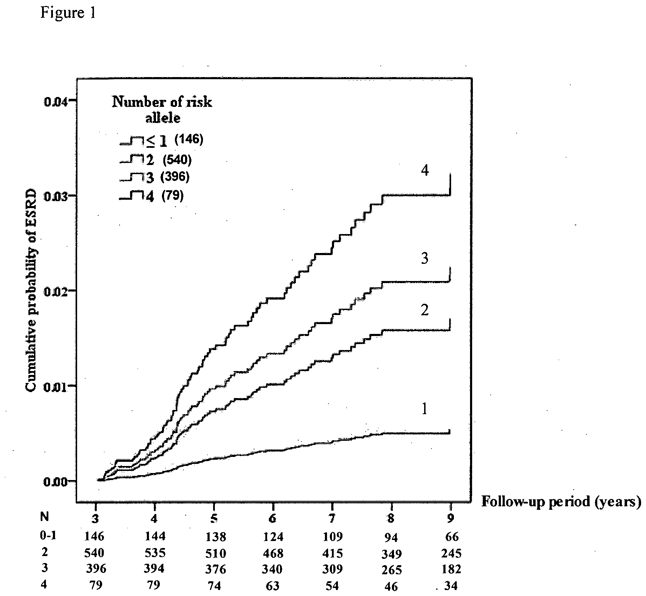

Figure 1. Cumulative probability of new-onset ESRD according to number of risk alleles, adjusted for mean values of the conventional risk factors (sex, age and duration of diabetes, systolic and diastolic blood pressure, HbA1c, total cholesterol, natural logarithm of triglycerides, eGFR, natural logarithm of AER, retinopathy (present/absent), the use of drugs (yes/no)). Three (rs3760106 with dominant model, rs7404928 with recessive model and rs4787733 with additive model) significant and independent (r² < 0.8) SNPs were selected to calculate the number of risk alleles for ESRD. The total number of carriers for the risk allele categories are indicated within the parentheses. The number of subjects exposed to risk at each follow-up period time point stratified by genotypes are indicated below the horizontal axis.

### Definitions

As used herein the terms "patient" and "subject" are not limited to human beings, but are intended to include all vertebrate animals in addition to human beings. In some embodiments disclosed herein, the terms "patient" and "subject" refer to any people or any ethnic groups in the word, such as Asian ancestry, including Chinese ancestry or descent. In other embodiments, the patient is a patient of type 2 diabetes, such as a patient of type 2 diabetes of Chinese ancestry.

As used herein the terms "genetic predisposition", "genetic susceptibility" and "susceptibility" all refer to the likelihood that an individual subject is suffering from, at risk for, or suspected of suffering from a particular disease, condition or disorder. For example, a subject with an increased susceptibility or predisposition will be more likely than average to develop a disease, while a subject with a decreased predisposition will be less likely than average to develop the disease.

As used herein, "gene" means any amount of nucleic acid material that is sufficient to encode a transcript or protein having the function desired. Thus, it includes, but is not limited to, genomic DNA, cDNA, RNA, and nucleic acid that are otherwise genetically engineered to achieve a desired level of expression under desired conditions. Accordingly, it includes fusion genes (encoding fusion proteins), intact genomic genes, and DNA sequences fused to heterologous promoters, operators, enhancers, and/or other transcription regulating sequences. The term refers to an entirety containing entire transcribed region and all regulatory regions of a gene. The transcribed region of a gene including all exon and intron sequences of a gene including alternatively spliced exons and introns so the transcribed region of a gene contains in addition to polypeptide encoding region of a gene also regulatory and 5' and 3' untranslated regions present in transcribed RNA.

As used herein, the terms "single nucleotide polymorphism", "SNP" and "genetic polymorphism" is a DNA sequence variation or a genetic variant that occurs when a nucleotide, e.g., adenine (A), thymine (T), cytosine (C), or guanine (G), in the genome sequence is altered to another nucleotide. SNPs are occasional variations in DNA sequence; the vast majority of the DNA sequence is identical among all humans, such as Accession Number RefSeq NM_002738 for the protein kinase C beta 1 gene of the invention. SNPs or other variants may also be found in genomic regions that do not contain genes. They represent a genomic hot spot responsible for the genetic variability among humans. The term SNP can be inter used with the term "genetic variant" or "variant" which is present at a particular genetic locus in at least one individual in a population and that differs from the corresponding reference type in the vast majority of the DNA sequence.

As used herein, the term "haplotype" refers to any combination of genetic variants or markers ("alleles") usually inherited together. A haplotype can comprise two or more alleles and the length of a genome region comprising a haplotype may vary from few hundred bases up to hundreds of kilobases. As it is recognized by those skilled in the art, the same haplotype can be described differently by determining the haplotype defining alleles from different nucleic acid strands. For example, the haplotype TTA defined by the SNP markers of this invention is the same as haplotype TAA in which the alleles are determined from the other strand. The haplotypes described herein are differentially present in the patients with increased risk of developing one or more of the aforementioned disease or complications. Therefore, these haplotypes have diagnostic value for risk assessment, diagnosis and prognosis of kidney disease and complications thereof. Detection of haplotypes can be accomplished by methods known in the art used for detecting nucleotides at polymorphic sites.

As used in this application, the singular form "a", "an" and "the" include plural references unless the context clearly dictates otherwise. In particular, where the indefinite article is used, the specification is to be understood as contemplating plurality as well as singularity, unless the context requires otherwise.

As used herein, the term "about" or "approximately" when used in conjunction with a number refers to any number within 1, 5 or 10% of the referenced number.

Reference throughout this specification to "one embodiment", or "an embodiment", or "in another embodiment", or "some embodiments", or "in certain embodiments" means that a particular referent feature, structure, or characteristic described in connection with the embodiment is included in at least one embodiment. Thus, the appearance of the phrases "in one embodiment", or "in an embodiment", or "in another embodiment", or "in some embodiments" in various places throughout this specification are not necessarily all referring to the same embodiment. Furthermore, the particular features, structures, or characteristics may be combined in any suitable manner in one or more embodiments.

### DETAILED DESCRIPTION OF THE EMBODIMENTS

One aspect disclosed herein provides a method for diagnosing a genetic predisposition in a subject for the diseases, disorders or conditions selected from the group consisting of end stage renal disease (ESRD) due to type 2 diabetes, ESRD due to hypertension in type 2 diabetes and ESRD due to type 1 diabetes. The method for diagnosing a genetic predisposition in a subject for the diseases, disorders or conditions comprises analyzing at least one polynucleotide to detect (i) a haplotype consisting of three variants or single nucleotide polymorphisms (SNPs) which are T allele at rs3760106,TT genotype at rs7404928, and A allele at rs4787733; and/or (ii) a haplotype consisting of four variants or single nucleotide polymorphisms (SNPs) which are T allele at rs3760106, G allele at rs2575390, TT genotype at rs7404928 and A allele at rs4787733in PKC-β1 gene in a sample obtained from the subject, wherein the presence of either or both haplotypes indicates the subject is suffering from, at risk for, or suspected of suffering from said diseases, disorders or conditions.

In one embodiment, the method comprises detecting a haplotype consisting of 3 variants or SNPs, which are T allele at rs3760106, TT genotype at rs7404928, and A allele at rs4787733 (i.e. TTA). In another embodiment, the method comprises detecting a haplotype consisting of 4 variants or SNPs which are which are T allele at rs3760106, G allele at rs2575390, TT genotype at rs7404928 and A allele at rs4787733, respectively.

Another aspect disclosed herein provides a kit or array for use in diagnosing a genetic predisposition in a subject for the diseases, disorders or conditions selected from the group consisting of end stage renal disease (ESRD) due to type 2 diabetes, ESRD due to hypertension in type 2 diabetes, ESRD due to type 1 diabetes, consisting of reagents for detecting (i) a haplotype consisting of three variants or single nucleotide polymorphisms (SNPs) which are T allele at rs3760106,TT genotype at rs7404928, and A allele at rs4787733; and/or (ii) a haplotype consisting of four variants or single nucleotide polymorphisms (SNPs) which are T allele at rs3760106, G allele at rs2575390, TT genotype at rs7404928 and A allele at rs4787733in a sample containing at least one polynucleotide obtained from said subject.

Although the numerical chromosomal position of a SNP may still change upon annotating the current human genome build the SNP identification information such as variable alleles and flanking nucleotide sequences assigned to a SNP will remain the same. Those skilled in the art will readily recognize that the analysis of the nucleotides present in one or more SNPs set forth herein in an individual's nucleic acid can be done by any method or technique capable of determining nucleotides present in a polymorphic site using the sequence information assigned in prior art to the rs IDs of the SNPs listed disclosed herein. As it is obvious in the art the nucleotides present in polymorphisms can be determined from either nucleic acid strand or from both strands.

Diagnostic kits (e.g. reagent kits) or arrays disclosed herein comprise reagents or materials, and optionally protocols or instructions for assessing one or more SNPs to make risk assessment, diagnosis or prognosis of a diabetes related complication and optimized therapeutic suggestions. Useful reagents and materials for kits include, but are not limited to PCR primers, hybridization probes and primers as described herein (e.g., labeled probes or primers), allele-specific oligonucleotides, reagents for genotyping SNP markers, reagents for detection of labeled molecules, restriction enzymes (e.g., for RFLP analysis), DNA polymerases, RNA polymerases, DNA ligases, marker enzymes, antibodies which bind to altered or to non-altered (native) a polypeptide, means for amplification of nucleic acids fragments from one or more SNPs selected from, means for analyzing the nucleic acid sequence of one or more diabetes-complication or ESRD related SNPs, or means for analyzing the sequence of one or more amino acid residues of polypeptides encoded by genes comprising such SNPs, etc. In one embodiment, a kit for diagnosing susceptibility to ESRD comprises primers and reagents for detecting the nucleotides present in SNP markers including rs3760106, rs7404928 and rs4787733, and optionally rs2575390, in PKC-β1 gene in individual's nucleic acid.

### Complications Associated with Diabetes

More than 90% of people diagnosed with diabetes, especially type 2 diabetes, carry a number of potential complications. Half of the people affected by diabetes die from complications resulting from the disease.

The complications involved, diagnosed or detected by the methods, kits or arrays of the present invention are end stage renal disease (ESRD) due to type 2 diabetes, ESRD due to hypertension in type 2 diabetes, and ESRD due to type 1 diabetes.

End-stage renal disease (ESRD) occurs when the kidneys cease to function, which ultimately leads to the need for a transplant or regular dialysis, both extremely costly procedures. Diabetes is responsible for about 50% of the cases of ESRD as a consequence of microvascular damage of the kidney.

### Preparation of Samples

The presence of genetic variants or SNPs in the PKC-β1 gene that may affect susceptibility to disease is determined by screening at least one nucleic acid sequence from an individual subject for such variants.

The nucleic acid sequence can be DNA or RNA. For the assay of genomic DNA, virtually any biological sample containing genomic DNA (e.g. not pure red blood cells) can be used. For example, and without limitation, genomic DNA can be conveniently obtained from blood, semen, saliva, tears, urine, fecal material, sweat, buccal cells, skin or hair. For assays using cDNA or mRNA, the target nucleic acid must be obtained from cells or tissues that express the target sequence. One preferred source and quantity of DNA is 3 to 30 ml of anticoagulated whole blood, since enough DNA can be extracted from leukocytes in such a sample to perform many repetitions of the analysis contemplated herein.

Many of the methods described herein require the amplification of DNA from target samples. This can be accomplished by any method known in the art but preferably is by the polymerase chain reaction (PCR). Optimization of conditions for conducting PCR must be determined for each reaction and can be accomplished without undue experimentation by one of ordinary skill in the art. In general, methods for conducting PCR can be found in Ausbel et al., eds., Short Protocols in Molecular Biology, 3.sup.rd ed., Wiley, 1995; and Innis et al., eds., PCR Protocols, Academic Press, 1990.

Other amplification methods include the ligase chain reaction (LCR) (see, Wu and Wallace, Genomics, 4:560-569, 1989; Landegren et al., Science, 241:1077-1080, 1988), transcription amplification (Kwoh et al., Proc. Natl. Acad. Sci. USA, 86:1173-1177, 1989), self-sustained sequence replication (Guatelli et al., Proc. Natl. Acad. Sci. USA, 87:1874-1878, 1990), and nucleic acid based sequence amplification (NASBA). The latter two amplification methods involve isothermal reactions based on isothermal transcription, which produces both single stranded RNA (ssRNA) and double stranded DNA (dsDNA) as the amplification products in a ratio of about 30 or 100 to 1, respectively.

### Polymorphism Detection

The polymorphism detection involves determining which form of the polymorphism is present in individuals for diagnostic or epidemiological purposes. The detection technology comprises PCR-based technology, or methods involving primer extension of multiplex products with detection by MALDI-TOF mass spectroscopy on a MassARRAY platform (Sequenom, San Diego, California). Several methods have been developed to detect known SNPs. Many of these assays have been reviewed by Landegren et al., Genome Res., 8:769-776, 1998, and will only be briefly reviewed here.

One type of assay has been termed an array hybridization assay, an example of which is the multiplexed allele-specific diagnostic assay (MASDA) (U.S. Pat. No. 5,834,181; Shuber et al., Hum. Molec. Genet., 6:337-347, 1997).

High throughput screening for SNPs that affect restriction sites can be achieved by Microtiter Array Diagonal Gel Electrophoresis (MADGE) (Day and Humphries, Anal. Biochem., 222:389-395, 1994).

Additional assays for SNPs depend on mismatch distinction by polymerases and ligases. This has allowed the use of PCR for the rapid detection of single base changes in DNA by using specifically designed oligonucleotides in a method variously called PCR amplification of specific alleles (PASA) (Sommer et al., Mayo Clin. Proc., 64:1361-1372 1989; Sarker et al., Anal. Biochem. 1990), allele-specific amplification (ASA), allele-specific PCR, and amplification refractory mutation system (ARMS) (Newton et al., Nuc. Acids Res., 1989; Nichols et al., Genomics, 1989; Wu et al., Proc. Natl. Acad. Sci. USA, 1989).

The joining by DNA ligases of two oligonucleotides hybridized to a target DNA sequence is quite sensitive to mismatches close to the ligation site, especially at the 3' end. This sensitivity has been utilized in the oligonucleotide ligation assay (OLA; Landegren et al., Science, 241:1077-1080, 1988) and the ligase chain reaction (LCR; Barany, Proc. Natl. Acad. Sci. USA, 88:189-193, 1991).

In another method for the detection of SNPs termed minisequencing, the target-dependent addition by a polymerase of a specific nucleotide immediately downstream (3') to a single primer is used to determine which allele is present (U.S. Pat. No. 5,846,710).

The above discussion of methods for the detection of SNPs is exemplary only and is not intended to be exhaustive.

### SNPs

In the present invention, PRKCB gene (accession number: NM_002738) is selected to determine the susceptibility to a disease, condition, or disorder in a subject, especially the diseases, disorders or conditions as described herein. The tagging SNPs comprising rs3760106, rs7404928 and rs4787733, and optionally rs2575390, are responsible for the susceptibility to the diseases, disorders or conditions in the subject. The methods, kits and arrays as described herein relate to detection of SNPs at genetic loci of rs3760106, rs2575390, rs7404928, and rs4787733. The methods, kits and arrays as described herein relate to detection of SNPs at the genetic loci comprising rs3760106, rs4787733, and rs7404928, and optionally rs2575390.

The SNP is associated with a genetic predisposition for a disease, condition or disorder selected from the group consisting of end stage renal disease due to non-insulin dependent diabetes, hypertension due to non-insulin dependent diabetes and end stage renal disease due to insulin dependent diabetes.

Although the methods or kits disclosed herein can be performed in sample obtained from a subject with or without diabetes, it is preferred to perform them in a subject who is suffering from Type 2 diabetes. The subject can be of any ethnic group, such as Asian, including Chinese descent.

After adjustment for age and sex, we surprised find that the risk for having the genetic susceptibility increases progressively and significantly with increasing number of risk genotypes or SNPs. Patients with 3 or 4 risk genotypes or SNPs had 1.5-2.0 fold increased risk for DKD or ESRD, compared to those patients with 0 or 1 risk genotype. In particular, the use of a haplotype consisting of the 3 variants (such as rs3760106, rs7404928, and rs4787733) indicates that individuals carrying the risk-conferring haplotype (such as TTA for rs3760106, rs7404928, and rs4787733) is associated with about 1.5 to 2.0 fold or more (for TTA for rs3760106, rs7404928, and rs4787733, about 1.89 fold) increased risk of end stage renal disease compared with those patients with 0 or 1 risk genotype, whilst carriers of the protective (such as CCG for rs3760106, rs7404928, and rs4787733) alleles at the 3 respective variants are protected against the development of diabetic kidney complications.

The risk of developing end stage renal disease increases with increasing number of risk alleles within the PRKCB gene. For example, the adjusted risk for end stage renal disease is more than 6 (95% CI, 2.00-18.31) for patients with 4 risk alleles (i.e. rs3760106, rs2575390, rs4787733, and rs7404928) compared with patients with 0 or 1 risk allele. Incidence of end stage renal disease among individuals carrying 4 risk alleles is increased compared with those carrying 0 or 1 risk allele. For example, incidence of end stage renal disease was 4.4 per 1000 person years (95% CI, 0.5-8.2) among individuals with 0 or 1 risk allele compared with 20.0 per 1000 person-years (95% CI, 8.8-31.1) in those carrying the 4 risk alleles (i.e. rs3760106, rs2575390, rs4787733, and rs7404928).

It is understood that one or more genotypes mentioned above can be used to develop arrays that are used in conjunction with other known clinical, biochemical and genetic for predicting the risk of diabetic complications including nephropathy and ESRD in diabetic patients of Chinese ancestry or other ethnic groups in the world, and these genotypes or equivalent arrays thereof can be used to identify at risk subjects for diabetes and/or diabetic ESRD for risk modification using a multifaceted approach including intensive monitoring, pharmacological and non-pharmacological therapy.

The present invention will be further described with the following Examples. blood, semen, saliva, tears, urine, fecal material, sweat, buccal cells, skin or hair. For assays using cDNA or mRNA, the target nucleic acid must be obtained from cells or tissues that express the target sequence. One preferred source and quantity of DNA is 3 to 30 ml of anticoagulated whole blood, since enough DNA can be extracted from leukocytes in such a sample to perform many repetitions of the analysis contemplated herein.

Many of the methods described herein require the amplification of DNA from target samples. This can be accomplished by any method known in the art but preferably is by the polymerase chain reaction (PCR). Optimization of conditions for conducting PCR must be determined for each reaction and can be accomplished without undue experimentation by one of ordinary skill in the art. In general, methods for conducting PCR can be found in Ausbel et al., eds., Short Protocols in Molecular Biology, 3.sup.rd ed., Wiley, 1995; and Innis et al., eds., PCR Protocols, Academic Press, 1990.

Other amplification methods include the ligase chain reaction (LCR) (see, Wu and Wallace, Genomics, 4:560-569, 1989; Landegren et al., Science, 241:1077-1080, 1988), transcription amplification (Kwoh et al., Proc. Natl. Acad. Sci. USA, 86:1173-1177, 1989), self-sustained sequence replication (Guatelli et al., Proc. Natl. Acad. Sci. USA, 87:1874-1878, 1990), and nucleic acid based sequence amplification (NASBA). The latter two amplification methods involve isothermal reactions based on isothermal transcription, which produces both single stranded RNA (ssRNA) and double stranded DNA (dsDNA) as the amplification products in a ratio of about 30 or 100 to 1, respectively.

### Polymorphism Detection

The polymorphism detection involves determining which form of the polymorphism is present in individuals for diagnostic or epidemiological purposes. The detection technology comprises PCR-based technology, or methods involving primer extension of multiplex products with detection by MALDI-TOF mass spectroscopy on a MassARRAY platform (Sequenom, San Diego, California). Several methods have been developed to detect known SNPs. Many of these assays have been reviewed by Landegren et al., Genome Res., 8:769-776, 1998, and will only be briefly reviewed here.

One type of assay has been termed an array hybridization assay, an example of which is the multiplexed allele-specific diagnostic assay (MASDA) (U.S. Pat. No. 5,834,181; Shuber et al., Hum. Molec. Genet., 6:337-347, 1997).

High throughput screening for SNPs that affect restriction sites can be achieved by Microtiter Array Diagonal Gel Electrophoresis (MADGE) (Day and Humphries, Anal. Biochem., 222:389-395, 1994).

Additional assays for SNPs depend on mismatch distinction by polymerases and ligases. This has allowed the use of PCR for the rapid detection of single base changes in DNA by using specifically designed oligonucleotides in a method variously called PCR amplification of specific alleles (PASA) (Sommer et al., Mayo Clin. Proc., 64:1361-1372 1989; Sarker et al., Anal. Biochem. 1990), allele-specific amplification (ASA), allele-specific PCR, and amplification refractory mutation system (ARMS) (Newton et al., Nuc. Acids Res., 1989; Nichols et al., Genomics, 1989; Wu et al., Proc. Natl. Acad. Sci. USA, 1989).

The joining by DNA ligases of two oligonucleotides hybridized to a target DNA sequence is quite sensitive to mismatches close to the ligation site, especially at the 3' end. This sensitivity has been utilized in the oligonucleotide ligation assay (OLA; Landegren et al., Science, 241:1077-1080, 1988) and the ligase chain reaction (LCR; Barany, Proc. Natl. Acad. Sci. USA, 88:189-193, 1991).

In another method for the detection of SNPs termed minisequencing, the target-dependent addition by a polymerase of a specific nucleotide immediately downstream (3') to a single primer is used to determine which allele is present (U.S. Pat. No. 5,846,710).

The above discussion of methods for the detection of SNPs is exemplary only and is not intended to be exhaustive. Those of ordinary skill in the art will be able to envision other methods for detection of SNPs that are within the scope and spirit of the present invention.

### SNPs

In the present invention, PRKCB gene (accession number: NM_002738) is selected to determine the susceptibility to a disease, condition, or disorder in a subject, especially the diseases, disorders or conditions as described herein. In an embodiment, the tagging SNPs comprising at least one of rs3760106, rs2575390, rs7404928 and rs4787733, are responsible for the susceptibility to the diseases, disorders or conditions in the subject. The methods, kits and arrays as described herein relate to detection of SNPs at genetic loci of rs3760106, rs2575390, rs7404928, and rs4787733. In one embodiment, the methods, kits and arrays as described herein relate to detection of SNPs at the genetic loci comprising at least one, at least two or at least three of: rs3760106, rs2575390, rs4787733, and rs7404928.

The SNP is associated with a genetic predisposition for a disease, condition or disorder comprising but not limited to non-insulin dependent diabetes, insulin dependent diabetes, end stage renal disease due to non-insulin dependent diabetes, hypertension due to non-insulin dependent diabetes, end stage renal disease due to hypertension of non-insulin dependent diabetes, atherosclerotic peripheral vascular disease due to non-insulin dependent diabetes, and end stage renal disease due to insulin dependent diabetes.

Although the methods or kits disclosed herein can be performed in sample obtained from a subject with or without diabetes, it is preferred to perform them in a subject who is suffering from Type 2 diabetes. The subject can be of any ethnic group, such as Asian, including Chinese descent.

After adjustment for age and sex, we surprised find that the risk for having the genetic susceptibility increases progressively and significantly with increasing number of risk genotypes or SNPs. Patients with 3 or 4 risk genotypes or SNPs had 1.5-2.0 fold increased risk for DKD or ESRD, compared to those patients with 0 or 1 risk genotype. In particular, the use of a haplotype consisting of the 3 variants (such as rs3760106, rs7404928, and rs4787733) indicates that individuals carrying the risk-conferring haplotype (such as TTA for rs3760106, rs7404928, and rs4787733) is associated with about 1.5 to 2.0 fold or more (for TTA for rs3760106, rs7404928, and rs4787733, about 1.89 fold) increased risk of end stage renal disease compared with those patients with 0 or 1 risk genotype, whilst carriers of the protective (such as CCG for rs3760106, rs7404928, and rs4787733) alleles at the 3 respective variants are protected against the development of diabetic kidney complications.

The risk of developing end stage renal disease increases with increasing number of risk alleles within the PRKCB gene. For example, the adjusted risk for end stage renal disease is more than 6 (95% CI, 2.00-18.31) for patients with 4 risk alleles (i.e. rs3760106, rs2575390, rs4787733, and rs7404928) compared with patients with 0 or 1 risk allele. Incidence of end stage renal disease among individuals carrying 4 risk alleles is increased compared with those carrying 0 or 1 risk allele. For example, incidence of end stage renal disease was 4.4 per 1000 person years (95% CI, 0.5-8.2) among individuals with 0 or 1 risk allele compared with 20.0 per 1000 person-years (95% CI, 8.8-31.1) in those carrying the 4 risk alleles (i.e. rs3760106, rs2575390, rs4787733, and rs7404928).

It is understood that one or more genotypes mentioned above can be used to develop arrays that are used in conjunction with other known clinical, biochemical and genetic for predicting the risk of diabetic complications including nephropathy and ESRD in diabetic patients of Chinese ancestry or other ethnic groups in the world, and these genotypes or equivalent arrays thereof can be used to identify at risk subjects for diabetes and/or diabetic ESRD for risk modification using a multifaceted approach including intensive monitoring, pharmacological and non-pharmacological therapy.

The present invention will be further described with the following Examples.

### EXAMPLE

### METHODS

### Study participants

Our cohort consists of 1338 unrelated patients with type 2 diabetes from the Hong Kong Diabetes Registry (HKDR) enrolled between 1995 and 1998. All subjects were of southern Han Chinese ancestry residing in Hong Kong. Patients with classical type 1 diabetes (defined as acute presentation with diabetic ketoacidosis, heavy ketonuria (>3+), or continuous requirement of insulin within 1 year of diagnosis) were excluded. In addition, patients with incomplete clinical information (N=4), follow-up period less than 3 years (N=145), requirement of dialysis at baseline (N=1), or had ESRD defined as estimated glomerular filtration rate (eGFR) less than 15 ml/min per 1.73 m² at enrollment (N=16) were excluded. The study design, ascertainment, inclusion criteria and phenotyping of the study subjects have been described ^{6 27}. For this analysis, a total of 1172 unrelated patients with type 2 diabetes were included (mean (±SD) age 56.0 ± 11.9 years, 41.3% male, disease duration 8.5 ± 6.7 years).

The validation cohort consisted of additional subjects recruited from the Hong Kong Diabetes Registry. Subjects included in the replication cohort were recruited into the Registry in an identical manner to the original cohort, but were recruited from 1998 onwards. Patients with chronic kidney disease at enrollment were excluded in the validation cohort. For the cross-sectional study on association between *PRKCB* genotype and quantitative measures of renal function, we recruited 1,892 unrelated Chinese type 2 diabetic patients from the inpatient database of Shanghai Diabetes Institute ²⁸. This study was approved by the Clinical Research Ethics Committee of the Chinese University of Hong Kong and the Ethics Committee of the Shanghai Jiaotong University. Written informed consent was obtained from all participants.

### Clinical studies and outcomes

All study participants were examined in the morning after an overnight fast. Anthropometric parameters including body weight and height, waist circumference and blood pressure were measured. Fasting blood samples were collected for measurement of plasma glucose, HbA_{1C}, and lipid profile (total cholesterol (TC), triglycerides (TG), HDL and LDL cholesterol). Hypertension was defined as blood pressure ≥ 130/85 mmHg or use of anti-hypertensive medications (not including angiotensin-coverting enzyme inhibitors (ACEIs) and angiotensin receptor blockers (ARBs)), or use of ACEIs or ARBs. A timed urine collection (4- or 24-hours) was used for measurement of urinary albumin excretion rate (AER). Glomerular filtration rate (eGFR) was estimated using the abbreviated formula developed by the Modification of Diet in Renal Disease (MDRD) further adjusted for the Chinese ethnicity: eGFR = 186 × [S_{CR} × 0.011]^{-1.154} × [age]^{-0.203} × [0.742 if female] × [1.233 if Chinese] where S_{CR} is serum creatinine expressed as µmol/l and 1.233 is the adjusting coefficient for Chinese population ²⁹. Use of medications, including oral blood glucose lowering agents and insulin treatment were also recorded for all study subjects. Anti-hypertensive medications included all classes of drugs that are indicated for hypertension, other than ACEI/ARB. Lipid-lowering drugs included statins and fibrates.

All clinical endpoints including hospital admissions and mortality were censored on 30^{th} July, 2005 according to the databases from the Hospital Authority Central Computer System, which records admissions to all public hospitals. These databases, including the Hong Kong Death Registry, were matched by a unique identification number, the Hong Kong Identity Card number, which is compulsory for all residents in Hong Kong and used by all government departments and major organizations. Using the International Classification of Diseases - 9^{th} Revision code, ESRD was defined as 1) death due to diabetes with renal manifestations (Code 250.4), chronic renal failure (Code 585) or unspecified renal failure (Code 586) or 2) nonfatal chronic renal failure (Code 585) or unspecified renal failure (Code 586) or 3) dialysis (ICD-9 procedure code: 39.95) or peritoneal dialysis (ICD-9 procedure code: 54.98) or 4) follow-up eGFR < 15 ml/min per 1.73 m².

In the validation cohort, we defined chronic kidney disease (CKD) as 1) death due to diabetes with renal manifestations (Code 250.4), chronic renal failure (Code 585) or unspecified renal failure (Code 586) (principle diagnosis only) or 2) nonfatal chronic renal failure (Code 585) or unspecified renal failure (Code 586) or 3) dialysis (ICD-9 procedure code: 39.95) or peritoneal dialysis (ICD-9 procedure code: 54.98) or 4) follow-up eGFR < 60 ml/min per 1.73 m². For the validation cohort, all clinical endpoints including hospital admissions and mortality were censored on 31^{st} December, 2008 according to the databases from the Hospital Authority Central Computer System, in identical manner as described earlier for the original cohort.

### Genotyping

Based on the Phase III HapMap database (HapMap release 27 [Feb. 2009], NCBI Build 36; dbSNP b126) specific for the Han Chinese (CHB) population, we selected 18 SNPs from a 388 kb region (chromosome 16:23,752,823 - 24,141,063 bp) spanning 2 kb upstream and downstream of PRKCB (accession number: NM_002738). Among these SNPs, 12 tagging SNPs (rs7404928, rs3785394, rs3785391, rs120908, rs4788423, rs4787733, rs198198, rs380932, rs429342, rs1015408, rs3785380, rs3785378) with r² < 0.8 and minor allele frequency (MAF) ≥ 0.05 were selected using the Tagger algorithm in the Haploview (v 4.1) program. Under the "common disease-common variant hypothesis", common diseases are hypothesized to have common causal variants (those with minor allele frequency (MAF) ≥ 0.05), so we focused on investigating the effects of common genetic variants in this study. Also, SNPs with r² ≥ 0.8 are considered redundant as 80% of their information overlapped, so selecting just one of them would be more cost-effective. In addition, we selected six SNPs (rs3760106, rs2575390, rs3900007, rs3900008, rs432998 and rs3729904) with suggestive evidence of association from previous studies ^{23 24}, so a total of 18 SNPs were genotyped in all study subjects in 2009 using genomic DNA.

Genotyping was performed at the McGill University and Genome Quebec Innovation Centre using primer extension of multiplex products with detection by MALDI-TOF mass spectroscopy on a Sequenom MassARRAY platform (San Diego, CA, USA). All SNPs were in Hardy-Weinberg equilibrium (*P* > 0.05), as assessed by the exact test of PLINK ³¹. The overall genotype call rate was 99.3%. Genotyping accuracy was demonstrated by showing > 99.6% overall concordance rate in 14 blinded duplicate samples. Samples from Shanghai were genotyped using MassARRAY iPLEX system (MassARRAY Compact Analyzer, Sequenom, San Diego, CA, USA).

### Bioinformatic analyses and functional annotation

To annotate the possible underlying functions of the genotyped loci within *PRKCB,* the data of chromatin structure, evolutionary conserved sequence, CpG islands, and cis-regulatory elements were assessed using the following available bioinformatics tools. The genomic locations (Ensembl Build 40, NBCI v36, hg18) of those SNPs were mapped from dbSNP130 and tracked on the University of California Santa Cruz human genome browser. The data of CpG islands, UW histone modification region ³² and *PRKCB* coding region were selected on the UCSC genome browser. The conserved sequence in the promoter region was identified by cisRED database ³³. The transcriptional regulatory modules within the gene were predicted by PReMOD³⁴.

### Statistical analysis

All data are expressed as percentage, mean ± SD or median (interquartile range), as appropriate. Triglycerides and AER were natural log-transformed due to skewed distributions. Between-group comparisons were performed by chi-squared test for categorical variables, and unpaired Student's t-test or the Wilcoxon Rank Sum test for continuous variables.

The relationships between *PRKCB* polymorphisms under additive, dominant and recessive genetic models and ESRD were tested by Cox proportional hazard regression model with adjustment for conventional risk factors at baseline including sex, age, duration of diabetes, systolic and diastolic blood pressure, HbA_{1c}, total cholesterol, natural logarithms of triglycerides and AER, eGFR, retinopathy (present/absent) and use of drugs (yes/no). Additive model assumes that the casual allele exerts an additive effect, so that carriers with 0, 1, or 2 risk alleles would have no, some and the most causal effect respectively. Dominant model assumes that all carriers with the casual allele (both homozygote and heterozygote) would have a causal effect, while recessive model assumes that only carriers with two causal alleles (homozygote) would have a causal effect. Hazard ratios (HRs) with 95% confidence intervals (CIs) were presented. We corrected for multiple comparisons of SNPs under additive, dominant and recessive genetic models using the permutation method rather than the false discovery rate approach. The largest test statistic obtained from the three models was chosen (MAX statistic) ³⁵. Since the distribution of the MAX statistic under the null hypothesis is unknown, experiment-wise significance was estimated from the empirical distribution of the MAX statistic after performing 10,000 permutations of genotypes for all 18 SNPs.

Pairwise linkage disequilibrium measures were computed in all samples using Haploview. Haplotype frequencies between patients with and without new-onset end stage renal disease were compared using haplotype-specific test implemented in Haploview. Hazard ratios (HRs) with 95% confidence intervals (CIs) were calculated for risk association of ESRD with various haplotypes. To assess interaction effects between pairwise SNPs on outcome variables, Cox proportional hazard regression model including the main and interaction effects of SNPs with covariates was applied.

The joint effect of the three *PRKCB* polymorphisms (rs3760106 with dominant model, rs7404928 with recessive model and rs4787733 with additive model) for ESRD risks were shown by Kaplan-Meier curves adjusted for conventional risk factors at baseline, for which the cumulative probability of new-onset events according to number of risk alleles was estimated. The significance of the trend was tested by Cox regression using the categories of risk allele carried as an independent variable.

All statistical analyses were performed using SAS v.9.1 (SAS Institute, Cary, NC, USA) or SPSS for Windows v.15 (SPSS, Chicago, IL, USA) unless specified otherwise. A two-tailed *P* value < 0.05 was considered statistically significant. We estimated the posterior study power using genetic power calculator ³⁶. Assuming dominant models with minor allele frequencies of 0.07, our sample size have 95% power to detect a minimal HR 2.25 for ESRD, at α level of 0.05.

### RESULTS

### Identification of genetic variants

We genotyped 18 SNPs spanning across the *PRKCB* gene in 1172 type 2 diabetic patients, including four SNPs in the promoter region, two SNPs in the coding exons and six SNPs in the non-coding intron region (Table 2). The gene structure and the location of SNPs are shown in eFigure 1. All SNPs were in modest linkage disequilibrium with r² <0.8 in our population except for four pairs of SNPs (rs3760106 and rs2575390; rs3900007 and rs3900008; rs3785394 and rs3785391; rs3785380 and rs3785378). The latter two pairs of SNPs demonstrated stronger LD in our population as compared to the HapMap CHB population (for rs3785394 and rs3785391, r² = 0.81 in present study and 0.78 in HapMap CHB data; for rs3785380 and rs3785378, r² = 0.94 in present study and 0.78 and HapMap CHB data).

### Clinical characteristics of study participants

During the study period, 90 patients developed ESRD. The mean follow-up period for ESRD was 7.9 ± 1.9 years. Patients with new-onset ESRD were older and had higher HbA_{1C}, TC, TG levels, LDL-C, systolic and diastolic blood pressure, AER, rates of hypertension and retinopathy and were more likely to be treated with insulin, ACEI and lipid-lowering drugs at baseline than those without. In addition, patients with new-onset ESRD had lower eGFR (Table 1).

### Association between variants of PRKCB and ESRD

The frequencies of the minor T-allele of rs3760106 and G-allele of rs2575390 (r² = 0.99) were markedly different between patients with (12.2%) and without incident ESRD (7%).

In the Cox-regression model after adjustment for conventional risk factors, four SNPs were significantly associated with ESRD (*P*<0.05) (Table 2). The T-allele at rs3760106 and the G-allele at the closely linked SNP rs2575390 (r² = 0.98) were strongly associated with increased risk for ESRD (HR (95% C.I.) = 2.25 (1.31 - 3.87), *P* = 0.0034 for rs3760106 in dominant model; HR (95% C.I.) = 2.26 (1.31 - 3.88), *P* = 0.0033 for rs2575390 in dominant model). We also observed nominal associations for ESRD with the common TT genotype of rs7404928 (HR (95% C.I.) = 1.59 (1.01 - 2.52), *P* = 0.0471 in recessive model) and the major A-allele of rs4787733 (HR (95% C.I.) = 1.78 (1.03 - 3.09), *P* = 0.0399 in additive model). The 2 SNPs rs3760106 (*P* = 0.0299) and rs2575390 (*P* = 0.0298) remained significant after correction for multiple comparisons.

Haplotype analyses of the three significant and independent SNPs (r² < 0.8) (rs3760106, rs7404928 and rs4787733) revealed similar effect size as compared with the single SNP associations (Table 3). Only haplotypes constructed from the risk-conferring (TTA) and protective (CCG) alleles conferred increased (HR (95% C.I.) = 1.89 (1.12 - 3.21), *P* = 0.0159) and decreased (HR (95% C.I.) = 0.38 (0.13 - 1.16), *P* = 0.0758) trends, respectively, for ESRD.

### Additive effects of variants of PRKCB and development of ESRD

We further examined possible two-way epistasis of *PRKCB* polymorphisms on ESRD, but failed to observe any evidence of interaction (data not shown). However, there were significantly increased risks for ESRD with increasing number of risk alleles (*P* = 0.0007 for ESRD) in the joint effect analysis of three (rs3760106 with dominant model, rs7404928 with recessive model and rs4787733 with additive model) significant and independent (r² < 0.8) SNPs (Figure 1 and table 4). The adjusted risk for ESRD was 6.044 (95% C.I. 2.00 - 18.31) in patients with four risk alleles compared to patients with one or no risk allele. Apart from presence of risk variants of PRKCB, other independent risk factors identified for development of ESRD were elevated HbA1c, decrease in eGFR, elevated log-transformed AER and presence of retinopathy (table 4). Among our cohort of subjects who were free of ESRD at baseline, new ESRD events occurred in 90 (7.7%) subjects during a maximum of 9-year follow-up, giving an annualized incidence of 9.7/1,000 person-years (95% CI = 7.7 - 11.7). The incidence was 4.4/1,000 person-years (95% CI = 0.5 - 8.2) for subjects carrying 0 or 1 risk alleles (12.3% of the cohort), compared to 20.0/1,000 (95% CI = 8.8 - 31.1) in those carrying 4 risk alleles (6.9% of the cohort).

### Validation of variants in PRKCB and development of diabetic kidney disease

In order to validate the association between *PRKCB* polymorphisms and diabetic kidney disease, we examined the effects of variants in *PRKCB* in additional subjects from the Hong Kong Diabetes Registry. All subjects in the validation cohort were recruited subsequent to the initial discovery cohort. The baseline characteristics of patients in the validation cohort were summarized in eTable 1. Patients in the validation cohort were older, with significantly shorter duration of diabetes and follow-up compared to the original cohort, and with significantly more patients treated with ACE inhibitors. As a result, a smaller proportion of the patients in this cohort developed ESRD during the follow-up period (253 subjects out of total 3677 subjects, 6.9%) compared with the original cohort. In order to examine the effects of variants of *PRKCB* on development of DKD in this cohort, we selected patients from this cohort with early-onset disease (defined as age of onset <45 years) who were free of chronic kidney disease at baseline and identified patients who progressed to chronic kidney disease during the follow-up period. The characteristics of this cohort of patients genotyped were summarized in eTable 1. Out of a total of 1049 patients, 151 (14.3%) developed CKD during the follow-up period. Both the T allele of the rs3760106 variant and the G allele of the rs2575390 variant were found to be significantly associated with development of CKD in this cohort, with HR of 1.68 (95% CI 1.1-2.57) and 1.62 (95% CI 1.07-2.47) respectively (eTable2). Although rs7404928 and the rs4787733 variants did not reach statistical significance for association with chronic kidney disease, the at-risk variants increased risk of developing renal dysfunction in the same direction using the same genetic model as in the original discovery cohort.

### Relationship between variants of PRKCB and eGFR

In order to better understand the relationship between genetic variants of *PRKCB* and development of renal dysfunction, we further examined the relationship between genetic variants in *PRKCB* and baseline renal function in an independent cross-sectional cohort of Chinese patients with type 2 diabetes recruited in Shanghai (eTable 3). We did not detect association between variants of *PRKCB* and baseline eGFR (p=0.66-0.82) or CKD (defined as eGFR < 60 ml/min per 1.73 m², n=195)(p=0.27-0.53) after adjustment for age, sex and BMI. This is similar to an analysis based on analysis of all cross-sectional data from baseline eGFR in our genotyped patients (data not shown).

### Functional annotation of the implicated genetic variants in PRKCB

We performed extensive bioinformatics analyses on the functional significance of the genetic variants identified. One of these variants, rs3760106, is known to lie in potential binding sites for the transcription factor Sp1 suggesting possible interaction between DNA and binding proteins ²³. Further bioinformatics analysis (summarized in eTable 4) suggest that rs3760106, rs2575390 and rs3900007 all lie within the conserved sequence of the *PRKCB* promoter, whist rs2575390 and rs3900007 both lie within histone modification sites (H3K27me3) and may thereby alter transcriptional activity of the gene. The rs7404928 and rs4787733 variants likewise lie within DNA regions with predicted functional significance which may alter the binding of regulatory factors and thereby lead to altered gene transcription (eTable 4).

### COMMENT

In this disclosure of patients with type 2 diabetes followed up for 8 years, we found that genetic variants of the *PRKCB* gene predicted development of incident ESRD independent of other known risk factors, with joint effects amongst the risk-conferring alleles. These associations persist despite correction for retinopathy, albuminuria, renal function, risk factor control and use of medications including ACEI at baseline. The strength of our study includes the relatively long duration of follow-up, detailed documentation of clinical parameters and drug use, as well as the use of ESRD as outcome measure for DKD. Our consistent results thus suggest that genetic variation in the *PRKCB* gene is an important determinant for the risk of developing DKD in patients with type 2 diabetes, such as Chinese patients.

. Our novel finding of an additive effect of increasing number of *PRKCB* variants in predicting diabetic kidney disease is unexpected, especially given that several of the variants were in non-coding regions. This may reflect effects of these variants on binding of other transcription factors, or other epigenetic effects, as predicted by our bioinformatics analyses. In this disclosure, our results indicate that the risk associations of the *PRKCB* polymorphisms with ESRD were independent of glycaemic control suggesting that other non-glycemic pathways may be implicated.

Taken together, our findings suggest that the risk association of renal disease with *PRKCB* may be mediated by interacting pathways including, but not limited to hyperglycaemia, and increased oxidative stress to cause tubular damage and interstitial fibrosis.

Our study was designed to examine the association between variants of *PRKCB* and development of renal dysfunction. Furthermore, given our biological understanding of the interaction between hyperglycaemia and activation of protein kinase C-β, one may expect the impact of variants of *PRKCB* to be specific for subjects with diabetes.

Given the important role of PKC-β in the pathogenesis of DKD and other diabetic vascular complications, a selective inhibitor of PKC-β, Ruboxistaurin (RBX), has been developed. This drug normalized haemodynamic changes, ECM accumulation, and histological features of glomerular damage associated with diabetes in several animal model ^{18 19 20}. In a Phase 2 clinical trial involving 123 type 2 diabetic patients with DKD treated optimally with ARB or ACEI, treatment with RBX further attenuated urinary TGF-β levels, decreased rate of loss of glomerular filtration rate and reduced proteinuria ^{22 57}. Preliminary analysis of markers of renal disease in patients with diabetic peripheral neuropathy participating in clinical trials of RBX also suggests improved renal parameters during the study period ^{58 59}. Given the phenotypic and genetic heterogeneity of complex diseases such as DKD, it would be of interest to examine the interaction between *PRKCB* genotype and clinical response to RBX, as recently demonstrated in the case for clinical response to ARB in subjects with the ACE insertion/deletion polymorphism ⁶⁰.

In summary, we found that genetic variants of the *PRKCB* gene predicted incident ESRD, independent of confounders notably, albuminuria, glycaemia, retinopathy and other risk factor control.

### REFERENCES

**1.** Ramachandran A, Ma RC, Snehalatha C. Diabetes in Asia. Lancet. Jan 30 2010;375(9712):408-418.
**2.** Chan JC, Malik V, Jia W, et al. Diabetes in Asia: epidemiology, risk factors, and pathophysiology. Jama. May 27 2009;301(20):2129-2140.
**3.** Yoon KH, Lee JH, Kim JW, et al. Epidemic obesity and type 2 diabetes in Asia. Lancet. Nov 11 2006;368(9548):1681-1688.
**4.** Yang W, Lu J, Weng J, et al. Prevalence of diabetes among men and women in China. N Engl J Med. Mar 25 2010;362(12):1090-1101.
**5.** Ritz E, Rychlik I, Locatelli F, Halimi S. End-stage renal failure in type 2 diabetes: A medical catastrophe of worldwide dimensions. Am J Kidney Dis. Nov 1999;34(5):795-808.
**6.** Yang X, So WY, Tong PC, et al. Development and validation of an all-cause mortality risk score in type 2 diabetes. Arch Intern Med. Mar 10 2008;168(5):451-457.
**7.** Wu AY, Kong NC, de Leon FA, et al. An alarmingly high prevalence of diabetic nephropathy in Asian type 2 diabetic patients: the MicroAlbuminuria Prevalence (MAP) Study. Diabetologia. Jan 2005;48(1):17-26.
**8.** Morrish NJ, Wang SL, Stevens LK, Fuller JH, Keen H. Mortality and causes of death in the WHO Multinational Study of Vascular Disease in Diabetes. Diabetologia. Sep 2001;44 Suppl 2:S14-21.
**9.** Karter AJ, Ferrara A, Liu JY, Moffet HH, Ackerson LM, Selby JV. Ethnic disparities in diabetic complications in an insured population. Jama. May 15 2002;287(19):2519-2527.
**10.** Yang XL, So WY, Kong AP, et al. End-stage renal disease risk equations for Hong Kong Chinese patients with type 2 diabetes: Hong Kong Diabetes Registry. Diabetologia. Oct 2006;49(10):2299-2308.
**11.** Yang XL, So WY, Kong AP, et al. Modified end-stage renal disease risk score for Chinese type 2 diabetic patients--the Hong Kong Diabetes Registry. Diabetologia. Jun 2007;50(6):1348-1350.
**12.** Soldatos G, Cooper ME. Diabetic nephropathy: important pathophysiologic mechanisms. Diabetes Res Clin Pract. Nov 13 2008;82 Suppl 1:S75-79.
**13.** Freedman BI, Bostrom M, Daeihagh P, Bowden DW. Genetic factors in diabetic nephropathy. Clin J Am Soc Nephrol. Nov 2007;2(6):1306-1316.
**14.** Sheetz MJ, King GL. Molecular understanding of hyperglycemia's adverse effects for diabetic complications. Jama. Nov 27 2002;288(20):2579-2588.
**15.** He Z, Ma RC, King GL. Role of Protein Kinase C Isoforms in Diabetic Vascular Dysfunction. In: Marso SP, Stem DM, eds. Diabetes and Cardiovascular Disease. Philadelphia, USA: Lippincott Williams & Wilkins; 2004:37-54.
**16.** Ma RC, Isshiki K, King GL. Protein kinase C and diabetic nephropathy: a perspective on its inhibition. In: Morgensen CE, ed. The Kidney and Hypertension in Diabetes Mellitus. 6th edition ed. Boston: Kluver Academic Press; 2004.
**17.** Noh H, King GL. The role of protein kinase C activation in diabetic nephropathy. Kidney Int Suppl. Aug 2007(106):S49-53.
**18.** Ishii H, Jirousek MR, Koya D, et al. Amelioration of vascular dysfunctions in diabetic rats by an oral PKC beta inhibitor. Science. May 3 1996;272(5262):728-731.
**19.** Koya D, Jirousek MR, Lin YW, Ishii H, Kuboki K, King GL. Characterization of protein kinase C beta isoform activation on the gene expression of transforming growth factor-beta, extracellular matrix components, and prostanoids in the glomeruli of diabetic rats. J Clin Invest. Jul 1 1997;100(1):115-126.
**20.** Koya D, Haneda M, Nakagawa H, et al. Amelioration of accelerated diabetic mesangial expansion by treatment with a PKC beta inhibitor in diabetic db/db mice, a rodent model for type 2 diabetes. Faseb J. Mar 2000;14(3):439-447.
**21.** Ohshiro Y, Ma RC, Yasuda Y, et al. Reduction of diabetes-induced oxidative stress, fibrotic cytokine expression, and renal dysfunction in protein kinase Cbeta-null mice. Diabetes. Nov 2006;55(11):3112-3120.
**22.** Tuttle KR, Bakris GL, Toto RD, McGill JB, Hu K, Anderson PW. The effect of ruboxistaurin on nephropathy in type 2 diabetes. Diabetes Care. Nov 2005;28(11):2686-2690.
**23.** Araki S, Ng DP, Krolewski B, et al. Identification of a common risk haplotype for diabetic nephropathy at the protein kinase C-betal (PRKCB1) gene locus. J Am Soc Nephrol. Aug 2003;14(8):2015-2024.
**24.** Araki S, Haneda M, Sugimoto T, et al. Polymorphisms of the protein kinase C-beta gene (PRKCB1) accelerate kidney disease in type 2 diabetes without overt proteinuria. Diabetes Care. Apr 2006;29(4):864-868.
**25.** Ng MC, Park KS, Oh B, et al. Implication of genetic variants near TCF7L2, SLC30A8, HHEX, CDKAL1, CDKN2A/B, IGF2BP2, and FTO in type 2 diabetes and obesity in 6,719 Asians. Diabetes. Aug 2008;57(8):2226-2233.
**26.** Luk AO, So WY, Ma RC, et al. Metabolic syndrome predicts new onset of chronic kidney disease in 5,829 patients with type 2 diabetes: a 5-year prospective analysis of the Hong Kong Diabetes Registry. Diabetes Care. Dec 2008;31(12):2357-2361.
**27.** Yang X, So WY, Kong AP, et al. Development and validation of stroke risk equation for Hong Kong Chinese patients with type 2 diabetes: the Hong Kong Diabetes Registry. Diabetes Care. Jan 2007;30(1):65-70.
**28.** Hu C, Wang C, Zhang R, et al. Association of genetic variants of NOS1AP with type 2 diabetes in a Chinese population. Diabetologia. Feb;53(2):290-298.
**29.** Ma YC, Zuo L, Chen JH, et al. Modified glomerular filtration rate estimating equation for Chinese patients with chronic kidney disease. J Am Soc Nephrol. Oct 2006;17(10):2937-2944.
**30.** Barrett JC, Fry B, Maller J, Daly MJ. Haploview: analysis and visualization of LD and haplotype maps. Bioinformatics. Jan 15 2005;21(2):263-265.
**31.** Purcell S, Neale B, Todd-Brown K, et al. PLINK: a tool set for whole-genome association and population-based linkage analyses. Am J Hum Genet. Sep 2007;81(3):559-575.
**32.** Sabo PJ, Hawrylycz M, Wallace JC, et al. Discovery of functional noncoding elements by digital analysis of chromatin structure. Proc Natl Acad Sci U S A. Nov 30 2004;101(48):16837-16842.
**33.** Robertson G, Bilenky M, Lin K, et al. cisRED: a database system for genome-scale computational discovery of regulatory elements. Nucleic Acids Res. Jan 1 2006;34(Database issue):D68-73.
**34.** Ferretti V, Poitras C, Bergeron D, Coulombe B, Robert F, Blanchette M. PReMod: a database of genome-wide mammalian cis-regulatory module predictions. Nucleic Acids Res. Jan 2007;35(Database issue):D122-126.
**35.** Sladek R, Rocheleau G, Rung J, et al. A genome-wide association study identifies novel risk loci for type 2 diabetes. Nature. Feb 22 2007;445(7130):881-885.
**36.** Purcell S, Cherny SS, Sham PC. Genetic Power Calculator: design of linkage and association genetic mapping studies of complex traits. Bioinformatics. Jan 2003;19(1):149-150.
**37.** Toyoda M, Suzuki D, Honma M, et al. High expression of PKC-MAPK pathway mRNAs correlates with glomerular lesions in human diabetic nephropathy. Kidney Int. Sep 2004;66(3):1107-1114.
**38.** Langham RG, Kelly DJ, Gow RM, et al. Increased renal gene transcription of protein kinase C-beta in human diabetic nephropathy: relationship to long-term glycaemic control. Diabetologia. Apr 2008;51(4):668-674.
**39.** Kramer HJ, Nguyen QD, Curhan G, Hsu CY. Renal insufficiency in the absence of albuminuria and retinopathy among adults with type 2 diabetes mellitus. Jama. Jun 25 2003;289(24):3273-3277.
**40.** MacIsaac RJ, Tsalamandris C, Panagiotopoulos S, Smith TJ, McNeil KJ, Jerums G. Nonalbuminuric renal insufficiency in type 2 diabetes. Diabetes Care. Jan 2004;27(1):195-200.
**41.** Fioretto P, Caramori ML, Mauer M. The kidney in diabetes: dynamic pathways of injury and repair. The Camillo Golgi Lecture 2007. Diabetologia. Aug 2008;51(8):1347-1355.
**42.** Fioretto P, Mauer M, Brocco E, et al. Patterns of renal injury in NIDDM patients with microalbuminuria. Diabetologia. Dec 1996;39(12):1569-1576.
**43.** Kelly DJ, Edgley AJ, Zhang Y, et al. Protein kinase C-beta inhibition attenuates the progression of nephropathy in non-diabetic kidney disease. Nephrol Dial Transplant. Jun 2009;24(6):1782-1790.
**44.** Chan JC, So WY, Yeung CY, et al. Effects of structured versus usual care on renal endpoint in type 2 diabetes: the SURE study: a randomized multicenter translational study. Diabetes Care. Jun 2009;32(6):977-982.
**45.** Song XY, Lee SY, Ma RC, et al. Phenotype-genotype interactions on renal function in type 2 diabetes: an analysis using structural equation modelling. Diabetologia. Aug 2009;52(8):1543-1553.
**46.** Sarafidis PA, Ruilope LM. Insulin resistance, hyperinsulinemia, and renal injury: mechanisms and implications. Am J Nephrol. 2006;26(3):232-244.
**47.** Forbes JM, Coughlan MT, Cooper ME. Oxidative stress as a major culprit in kidney disease in diabetes. Diabetes. Jun 2008;57(6): 1446-1454.
**48.** Etoh T, Inoguchi T, Kakimoto M, et al. Increased expression of NAD(P)H oxidase subunits, NOX4 and p22phox, in the kidney of streptozotocin-induced diabetic rats and its reversibity by interventive insulin treatment. Diabetologia. Oct 2003;46(10):1428-1437.
**49.** Asaba K, Tojo A, Onozato ML, et al. Effects of NADPH oxidase inhibitor in diabetic nephropathy. Kidney Int. May 2005;67(5):1890-1898.
**50.** Gorin Y, Block K, Hernandez J, et al. Nox4 NAD(P)H oxidase mediates hypertrophy and fibronectin expression in the diabetic kidney. J Biol Chem. Nov 25 2005;280(47):39616-39626.
**51.** Gao L, Mann GE. Vascular NAD(P)H oxidase activation in diabetes: a double-edged sword in redox signalling. Cardiovasc Res. Apr 1 2009;82(1):9-20.
**52.** Kottgen A, Glazer NL, Dehghan A, et al. Multiple loci associated with indices of renal function and chronic kidney disease. Nat Genet. May 2010;42(5): 376-84.
**53.** Kottgen A, Pattaro C, Boger CA, et al. New loci associated with kidney function and chronic kidney disease. Nat Genet. May 2010;42(5):376-384.
**54.** Chambers JC, Zhao J, Terracciano CM, et al. Genetic variation in SCN10A influences cardiac conduction. Nat Genet. Feb 2010;42(2):149-152.
**55.** Dupuis J, Langenberg C, Prokopenko I, et al. New genetic loci implicated in fasting glucose homeostasis and their impact on type 2 diabetes risk. Nat Genet. Feb;42(2):105-116.
**56.** Rothman KJ, Greenland S. Causation and causal inference in epidemiology. Am J Public Health. 2005;95 Suppl 1:S144-150.
**57.** Gilbert RE, Kim SA, Tuttle KR, et al. Effect of ruboxistaurin on urinary transforming growth factor-beta in patients with diabetic nephropathy and type 2 diabetes. Diabetes Care. Apr 2007;30(4):995-996.
**58.** Tuttle KR, Bastyr EJ, McGill JB, Cheng CL, Anderson PW. Albuminuria and kidney function in a long-term study of ruboxistaurin for diabetic nephropathy. J Am Soc Nephrol. 2007;18:48A.
**59.** Tuttle KR. Protein kinase C-beta inhibition for diabetic kidney disease. Diabetes Res Clin Pract. Nov 13 2008;82 Suppl 1:S70-74.
**60.** Parving HH, de Zeeuw D, Cooper ME, et al. ACE gene polymorphism and losartan treatment in type 2 diabetic patients with nephropathy. J Am Soc Nephrol. Apr 2008;19(4):771-779.

**TABLE 1. Clinical characteristics and biochemical profile at baseline stratified according to the progression to ESRD in Chinese type 2 diabetic patients.**

| | **ESRD** | | |
|---|---|---|---|
| | **No** | **Yes** | **P-value** |
| **N** | 1082 | 90 | |
| **Clinical characteristics** | | | |
| **Sex (male / female)** | 437 / 645 | 47 / 43 | 0.028 |
| **Age (years)** | 55**.**7 ± 11.9 | 60.4 ± 10.7 | <0.0001 |
| **Age Onset (years)** | 47**.**2 ± 12 | 51.6 ± 11.3 | 0.001 |
| **Duration of diabetes (years)** | 8**.**5 ± 6**.**8 | 8.8 ± 6.3 | 0.633 |
| **BMI (kg/m²)** | 24**.**8 ± 3**.**7 | 24.9 ± 4 | 0.771 |
| **Waist circumference (cm)** | | | |
| **Male** | 87**.**5 ± 9**.**0 | 89.3 ± 10.3 | 0.207 |
| **Female** | 83.0 ± 9.7 | 83.4 ± 11.1 | 0.776 |
| **HbA_{1C} (%)** | 7**.**9 ± 1.8 | 8.6 ± 2.2 | 0.002 |
| **Total cholesterol (mg/dL)** | 208**.**5 ± 42**.**5 | 235.5 ± 65.6 | 0.001 |
| **Triglycerides (mg/dL)** | 115.0 (79.7 - 177.0) | 150.4 (106.2 - 221.2) | <0.0001 |
| **HDL-cholesterol (mg/dL)** | 50**.**2 ± 15.4 | 46.3 ± 15.4 | 0.212 |
| **LDL-cholesterol (mg/dL)** | 131**.**3 ± 37.1 | 146.7 ± 50.2 | 0.015 |
| **Systolic blood pressure (mmHg)** | 134.9 ± 20.6 | 152.1 ± 26.6 | <0.0001 |
| **Diastolic blood pressure (mmHg)** | 78.3 ± 10.8 | 83.8 ± 12.7 | <0.0001 |
| **Hypertension % (N)** | 63% (682) | 88% (79) | <0.0001 |
| **Retinopathy** % **(N)** | 24% (260) | 62% (56) | <0.0001 |
| **AER (µg/min)** | 14.3 (8 - 62.4) | 1008 (269.4 - 2033.1) | <0.0001 |
| **eGFR (min/ml per 1.73 m²)** | 114.8 ± 33.9 | 70.4 ± 32.6 | <0.0001 |
| **Treatment** | | | |
| **Lipid lowering % (N)** | 6% (65) | 12% (11) | 0.011 |
| **Blood pressure anti-hypertensive % (N)** | 22% (238) | 48% (43) | <0.0001 |
| **ACE inhibitor % (N)** | 8% (87) | 20% (18) | <0.0001 |
| **Oral glucose lowering % (N)** | 50% (541) | 52% (47) | 0.661 |
| **Insulin treatment % (N)** | 15% (162) | 27% (24) | 0.006 |

| | | | |
|---|---|---|---|
| Data are shown as N, mean ± SD or median (interquartile range). | | | |

**TABLE 2. Genotype distributions of PRKCB SNPs and hazard ratio of PRKCB polymorphisms for risk of ESRD in Chinese type 2 diabetic patients.**

| | | | **ESRD** | **No ESRD** | **Additive** | | **Dominant** | | **Recessive** | |
|---|---|---|---|---|---|---|---|---|---|---|
| **SNP** | **Location** | **Risk / non-ris k allele** | **RR/RN/NN genotype frequencies** | **RR/RN/NN genotype frequencies** | **P** | **HR (95% C. I.)** | **P** | **HR (95% C. I.)** | **P** | **HR (95% C. I.)** |
| rs3760106 | Promoter | T/C | 0.000 / 0.244 / 0.756 | 0.004 / 0.132 / 0.865 | 0.0035 | 2.22 (1.30 - 3.80) | *0.0034^{a}* | 2.25 (1.31 - 3.87) | 0.9861 | -- |
| rs2575390 | Promoter | G/C | 0.000 / 0.244 / 0.756 | 0.004 / 0.132 / 0.865 | 0.0034 | 2.23 (1.30 - 3.81) | *0.0033^{a}* | 2.26 (1.31 - 3.88) | 0.9862 | -- |
| rs3900007 | Promoter | C/T | 0.111 / 0.544 / 0.344 | 0.111 / 0.449 / 0.439 | 0.8464 | 1.03 (0.74 - 1.45) | 0.4305 | 1.21 (0.76 - 1.93) | *0.4297* | 0.73 (0.34 - 1.58) |
| rs3900008 | Promoter | C/T | 0.111 / 0.544 / 0.344 | 0.113 / 0.449 / 0.438 | 0.8518 | 1.03 (0.74 - 1.45) | 0.4309 | 1.21 (0.76 - 1.93) | *0.4231* | 0.73 (0.34 - 1.57) |
| rs7404928 | Intron | T/C | 0.461 / 0.393 / 0.146 | 0.437 / 0.451 / 0.112 | 0.1855 | 1.27 (0.89 - 1.80) | 0.8231 | 0.93 (0.48 - 1.78) | *0.0471* | 1.59 (1.01 - 2.52) |
| rs3785394 | Intron | T/C | 0.382 / 0.472 / 0.146 | 0.397 / 0.474 / 0.130 | 0.4977 | 1.13 (0.80 - 1.58) | 0.9162 | 1.04 (0.53 - 2.04) | *0.3838* | 1.23 (0.78 - 1.94) |
| rs3785391 | Intron | T/C | 0.467 / 0.433 / 0.100 | 0.456 / 0.443 / 0.102 | 0.5749 | 1.10 (0.78 - 1.55) | *0.4614* | 1.36 (0.60 - 3.10) | 0.7660 | 1.07 (0.69 - 1.67) |
| rs120908 | Intron | C/A | 0.400 / 0.444 / 0.156 | 0.314 / 0.490 / 0.196 | 0.2295 | 1.21 (0.89 - 1.64) | *0.1230* | 1.66 (0.87 - 3.15) | 0.5766 | 1.14 (0.72 - 1.79) |
| rs4788423 | Intron | C/T | 0.303 / 0.416 / 0.281 | 0.215 / 0.499 / 0.286 | 0.2669 | 1.20 (0.87 - 1.66) | 0.9524 | 0.99 (0.59 - 1.64) | *0.0542* | 1.63 (0.99 - 2.67) |
| rs4787733 | Intron | A/G | 0.811 / 0.189 / 0.000 | 0.769 / 0.221 / 0.010 | *0.0399* | 1.78 (1.03 - 3.09) | 0.9847 | -- | 0.0609 | 1.74 (0.98 - 3.12) |
| rs198198 | Intron | T/A | 0.116 / 0.488 / 0.395 | 0.168 / 0.494 / 0.338 | 0.8963 | 1.02 (0.73 - 1.43) | *0.5953* | 1.14 (0.71 - 1.83) | 0.6243 | 0.84 (0.42 - 1.68) |
| rs380932 | Intron | G/T | 0.044 / 0.567 / 0.389 | 0.102 / 0.452 / 0.446 | 0.4396 | 1.16 (0.80 - 1.68) | *0.2150* | 1.35 (0.84 - 2.17) | 0.5595 | 0.73 (0.26 - 2.07) |
| rs432998 | Exon | G/A | 0.100 / 0.556 / 0.344 | 0.121 / 0.456 / 0.423 | 0.6454 | 1.09 (0.76 - 1.55) | *0.4078* | 1.22 (0.76 - 1.97) | 0.7128 | 0.86 (0.39 - 1.90) |
| rs429342 | Intron | G/C | 0.022 / 0.200 / 0.778 | 0.009 / 0.206 / 0.785 | 0.1666 | 1.41 (0.87 - 2.28) | *0.0881* | 1.63 (0.93 - 2.87) | 0.7543 | 0.72 (0.09 - 5.84) |
| rs3729904 | Exon | A/G | 0.000 / 0.156 / 0.844 | 0.009 / 0.153 / 0.837 | 0.1354 | 1.56 (0.87 - 2.78) | *0.0823* | 1.74 (0.93 - 3.25) | 0.9823 | -- |
| rs1015408 | Intron | T/A | 0.618 / 0.348 / 0.034 | 0.625 / 0.333 / 0.043 | 0.9745 | 1.01 (0.68 - 1.50) | *0.8978* | 1.08 (0.33 - 3.60) | 0.9886 | 1.00 (0.62 - 1.60) |
| rs3785380 | Intron | T/C | 0.056 / 0.289 / 0.656 | 0.030 / 0.263 / 0.708 | 0.4979 | 1.16 (0.76 - 1.76) | 0.6497 | 1.12 (0.69 - 1.80) | *0.3511* | 1.76 (0.54 - 5.82) |
| rs3785378 | Intron | C/T | 0.056 / 0.292 / 0.652 | 0.033 / 0.268 / 0.699 | 0.5536 | 1.13 (0.75 - 1.72) | 0.6912 | 1.10 (0.68 - 1.77) | *0.4216* | 1.63 (0.50 - 5.37) |

| | | | | | | | | | | |
|---|---|---|---|---|---|---|---|---|---|---|
| Hazard ratios refer to the risk-conferring alleles. *P* values were calculated from Cox proportional hazard regression adjusted for conventional risk factors (sex, age and duration of diabetes, systolic and diastolic blood pressure, HbA1c, total cholesterol, natural logarithm of triglycerides, eGFR, natural logarithm of AER, retinopathy (present/absent), the use of drugs (yes/no)) at baseline. Italic refers to the genetic model considered in MAX statistics. ^{a} significant SNP after corrected for multiple comparisons. | | | | | | | | | | |

**Table 3 Association between haplotype consisting of three significant SNPs in PRKCB region and ESRD endpoint in Chinese type 2 diabetic patients**

| **Haplotype frequencies** | | | | | | |
|---|---|---|---|---|---|---|
| **rs3760106** | **rs7404928** | **rs4787733** | **ESRD** | **No ESRD** | **OR (95% CI)** | *P* |
| C | T | A | 0.485 | 0.535 | 0.82 (0.60 - 1.11) | 0.1996 |
| C | C | A | 0.304 | 0.280 | 1.12 (0.81 - 1.57) | 0.4879 |
| C | T | G | 0.070 | 0.069 | 1.02 (0.56 - 1.84) | 0.9553 |
| T | T | A | 0.097 | 0.054 | 1.89 (1.12 - 3.21) | 0.0159 |
| C | C | G | 0.018 | 0.046 | 0.38 (0.13 - 1.16) | 0.0758 |
| T | C | A | 0.019 | 0.011 | 1.78 (0.57 - 5.51) | 0.3274 |

| | | | | | | |
|---|---|---|---|---|---|---|
| Haplotypes were compared between patients with and those without new-onset of ESRD by haplotype-specific tests (1 df) using Haploview. "TTA" and "CCG" are constructed from the risk and protective alleles for rs3760106, rs7404928 and rs4787733, respectively. | | | | | | |

**Table 4 Cox-regression analysis with hazard ratios of predictors for ESRD in Type 2 diabetes. Three (rs3760106 with dominant model, rs7404928 with recessive model and rs4787733 with additive model) significant and independent (r² < 0.8) SNPs were selected to calculate the number of risk alleles for ESRD.**

| | | **ESRD** | |
|---|---|---|---|
| | **Increment Unit** | *P* | **HR (95% CI)** |
| **Sex (male / female)** | per 1 % | 0.4281 | 0.82 (0.49 - 1.35) |
| **Age (years)** | per year | 0.6355 | 0.99 (0.97 - 1.02) |
| **Duration of diabetes (years)** | per year | 0.7713 | 1.01 (0.96 - 1.05) |
| **Systolic blood pressure (mmHg)** | per each 1-mm Hg | 0.5578 | 1.00 (0.98 - 1.01) |
| **Diastolic blood pressure (mmHg)** | per each 1-mm Hg | 0.2115 | 1.02 (0.99 - 1.05) |
| **Total cholesterol (mg/dL)** | per each 1-mg/dL | 0.5706 | 1.06 (0.87 - 1.29) |
| **log-transformed Triglycerides (mg/dL)** | per each 1-mg/dL | 0.0816 | 0.70 (0.47 - 1.05) |
| **HbA_{1C} (%)** | per 1 % | 0.0001 | 1.23 (1.11 - 1.37) |
| **eGFR (min/ml per 1.73 m²)** | per each 1-min/ml per 1.73 m² | 4.42 × 10⁻⁹ | 0.97 (0.96 - 0.98) |
| **log-transformed AER (µg/min)** | Per each 1-µg/min | 1.72 × 10⁻¹¹ | 1.81 (1.52 - 2.15) |
| **Retinopathy (%)** | per 1 % | 0.0139 | 1.87 (1.14 - 3.08) |
| **Lipid lowering (%)** | per 1 % | 0.6932 | 1.15 (0.57 - 2.34) |
| **Blood pressure anti-hypertensive (%)** | per 1 % | 0.8802 | 1.04 (0.61 - 1.79) |
| **ACE inhibitor (%)** | per 1 % | 0.7689 | 0.91 (0.48 - 1.73) |
| **Oral glucose lowering (%)** | per 1 % | 0.3319 | 1.31 (0.76 - 2.27) |
| **Insulin treatment (%)** | per 1 % | 0.6596 | 1.13 (0.65 - 1.99) |
| **Number of risk alleles** | | | |
| **0- 1** | | Reference group | |
| **2** | | 0.0186 | 3.18 (1.21 - 8.35) |
| **3** | | 0.0048 | 4.21 (1.55 - 11.42) |
| **4** | | 0.0015 | 6.04 (2.00 - 18.31) |

## Claims

1. A method for diagnosing a genetic predisposition in a subject for a disease, disorder or condition comprising a diabetic kidney complication selected from the group consisting of end stage renal disease (ESRD) due to type 2 diabetes, ESRD due to hypertension in type 2 diabetes, and ESRD due to type 1 diabetes, comprising detecting in a sample of the subject:
(i) a haplotype consisting of three variants or single nucleotide polymorphisms (SNPs) which are T allele at rs3760106, A allele at rs4787733, and TT genotype at rs7404928; and/or
(ii) a haplotype consisting of four variants or single nucleotide polymorphisms (SNPs) which are T allele at rs3760106, G allele at rs2575390, TT genotype at rs7404928, and A allele at rs4787733,
wherein the presence of either or both haplotypes indicates that the subject is suffering from, at risk for, or suspected of suffering from said disease, disorder or condition.

2. The method of claim 1 wherein the sample is selected from the group consisting of blood, semen, saliva, tears, urine, fecal material, sweat, buccal cells, skin, hair or tissue containing genomic DNA or expressing the target sequence.

3. The method of claims 1 or 2, wherein the subject is suffering from Type 2 Diabetes.

4. A kit or an array for use in diagnosing a genetic predisposition in a subject for a disease, disorder or condition comprising a diabetic kidney complication selected from the group consisting of end stage renal disease (ESRD) due to type 2 diabetes, ESRD due to hypertension in type 2 diabetes, and ESRD due to type 1 diabetes, wherein the kit or array consists of allele-specific oligonucleotides for detecting:
(i) a haplotype consisting of three variants or single nucleotide polymorphisms (SNPs) which are T allele at rs3760106, A allele at rs4787733, and TT genotype at rs7404928; and/or
(ii) a haplotype consisting of four variants or single nucleotide polymorphisms (SNPs) which are T allele at rs3760106, G allele at rs2575390, TT genotype at rs7404928, and A allele at rs4787733
in a sample containing at least one polynucleotide obtained from said subject.

## Patentansprüche

1. Ein Verfahren zum Dianostizieren einer genetischen Prädisposition in einem Individuum für eine Erkrankung, Störung oder einen Krankheitszustand, die oder der eine diabetische Nierenkomplikation beinhaltet, ausgewählt aus der Gruppe, die aus terminaler Niereninsuffizienz (ESRD) aufgrund Typ-2-Diabetes, ESRD aufgrund Hypertonie bei Typ-2-Diabetes and ESRD aufgrund Typ-1-Diabetes besteht, wobei das Verfahren den Nachweis der Folgenden in einer Probe des Individuums beinhaltet:
(i) eines Haplotyps, der aus drei Varianten oder Einzelnucleotidpolymorphismen (SNPs) besteht, die T-Allel bei rs3760106, A-Allel bei rs4787733 und TT-Genotyp bei rs7404928 sind; und/oder
(i) eines Haplotyps, der aus vier Varianten oder Einzelnucleotidpolymorphismen (SNPs) besteht, die T-Allel bei rs3760106, G-Allel bei rs2575390, TT-Genotyp bei rs7404928 undA-Allel bei rs4787733 sind,
wobei das Vorhandensein von einem der oder von beiden Haplotypen darauf hinweist, dass das Individuum an der Erkrankung, Störung oder dem Krankheitszustand leidet, bei dem Individuum ein Risiko dafür besteht oder der Verdacht besteht, dass das Individuum daran leidet.

2. Das Verfahren gemäß Anspruch 1, wobei die Probe aus der Gruppe ausgewählt ist, die aus Blut, Samen, Speichel, Tränen, Urin, Fäzesmaterial, Schweiß, Bukkalzellen, Haut, Haar oder Gewebe besteht, die genomische DNA enthalten oder die Zielsequenz exprimieren.

3. Das Verfahren gemäß Ansprüchen 1 oder 2, wobei das Individuum an Typ-2-Diabetes leidet.

4. Ein Kit oder eine Anordnung zur Verwendung beim Diagnostizieren einer genetischen Prädisposition in einem Individuum für eine Erkrankung, Störung oder einen Krankheitszustand, die oder der eine diabetische Nierenkomplikation beinhaltet, ausgewählt aus der Gruppe, die aus terminaler Niereninsuffizienz (ESRD) aufgrund Typ-2-Diabetes, ESRD aufgrund Hypertonie bei Typ-2-Diabetes and ESRD aufgrund Typ-1-Diabetes besteht, wobei das Kit oder die Anordnung aus allelspezifischen Oligonucleotiden zum Nachweis der Folgenden besteht:
(i) eines Haplotyps, der aus drei Varianten oder Einzelnucleotidpolymorphismen (SNPs) besteht, die T-Allel bei rs3760106, A-Allel bei rs4787733 und TT-Genotyp bei rs7404928 sind; und/oder
(ii) eines Haplotyps, der aus vier Varianten oder Einzelnucleotidpolymorphismen (SNPs) besteht, die T-Allel bei rs3760106, G-Allel bei rs2575390, TT-Genotyp bei rs7404928 undA-Allel bei rs4787733 sind,
in einer von dem Individuum erhaltenen Probe, die mindestens ein Polynucleotid enthält.

## Revendications

1. Méthode destinée à diagnostiquer chez un sujet une prédisposition génétique à une maladie, un trouble ou une condition comprenant une complication rénale du diabète sélectionnée parmi le groupe consistant en maladie rénale en stade terminal (ESRD) due au diabète de type 2, ESRD due à l'hypertension dans le diabète de type 2, et ESRD due au diabète de type 1, comprenant détecter dans un échantillon du sujet :
(i) un haplotype consistant en trois variantes ou polymorphismes d'un seul nucléotide (SNP) qui sont l'allèle T à rs3760106, l'allèle A à rs4787733 et le génotype TT à rs7404928 ; et/ou
(ii) un haplotype consistant en quatre variantes ou polymorphismes d'un seul nucléotide (SNP) qui sont l'allèle T à rs3760106, l'allèle G à rs2575390, le génotype TT à rs7404928 et l'allèle A à rs4787733,
où la présence de l'un ou des deux haplotypes indique que le sujet souffre, est à risque de souffrir ou soupçonné souffrir de ladite maladie, dudit trouble ou de ladite condition.

2. Méthode selon la revendication 1, dans laquelle l'échantillon est sélectionné parmi le groupe consistant en sang, sperme, salive, larmes, urine, matière fécale, transpiration, cellules buccales, peau, poil ou tissu contenant de l'ADN génomique ou exprimant la séquence cible.

3. Méthode selon les revendications 1 ou 2, dans laquelle le sujet souffre de diabète de type 2.

4. Kit ou biopuce à utiliser pour diagnostiquer chez un sujet une prédisposition génétique à une maladie, un trouble ou une condition comprenant une complication rénale du diabète sélectionnée parmi le groupe consistant en maladie rénale en stade terminal (ESRD) due au diabète de type 2, ESRD due à l'hypertension dans le diabète de type 2, et ESRD due au diabète de type 1, où le kit ou la biopuce consiste en oligonucléotides allèle-spécifiques pour détecter :
(i) un haplotype consistant en trois variantes ou polymorphismes d'un seul nucléotide (SNP) qui sont l'allèle T à rs3760106, l'allèle A à rs4787733 et le génotype TT à rs7404928 ; et/ou
(ii) un haplotype consistant en quatre variantes ou polymorphismes d'un seul nucléotide (SNP) qui sont l'allèle T à rs3760106, l'allèle G à rs2575390, le génotype TT à rs7404928 et l'allèle A à rs4787733,
dans un échantillon contenant au moins un polynucléotide obtenu dudit sujet.
